# EUROPEAN PATENT APPLICATION

(11) **EP 2 792 359 A1**
(43) Date of publication of application: **22.10.2014**
(21) Application number: 13002077.9
(22) Date of filing: 19.04.2013
(51) Int. Cl.: A61K 31/496, A61P 21/00, A61P 25/14, A61P 43/00

(54) **Treatment of L-DOPA-induced dyskinesia with OPC-14523 or OPC-34712**

(71) Applicant: Merz Pharma GmbH & Co. KGaA, 60318 Frankfurt am Main (DE)
(72) Inventor: Tiikkainen, Pekka, 60318 Frankfurt am Main (DE); Franke, Lutz, 61118 Bad Vilbel (DE)
(74) Representative: Ricker, Mathias

(57) **Abstract**

The present invention relates to the efficient treatment of an individual afflicted with L-DOPA-induced dyskinesia, which condition typically arises as a consequence of long-term treatment with L-DOPA therapy in Parkinson patients.

## Description

### FIELD OF THE INVENTION

The present invention relates to the efficient treatment of an individual afflicted with L-DOPA-induced dyskinesia, which condition typically arises as a consequence of long-term treatment with L-DOPA therapy in Parkinson patients

### BACKGROUND OF THE INVENTION

This invention relates to an innovative method of treating patients afflicted with L-DOPA-induced dyskinesia, which condition may arise as a consequence of long-term treatment with L-DOPA therapy in Parkinson patients.

Parkinsonian symptoms are characterized by slowness of movement (bradykinesia/akinesia), rigidity and/or tremor. These symptoms may have an idiopathic, toxic, traumatic or genetic origin (e.g., in Parkinson's disease (PD)) or may also occur as a consequence of treatment, e.g., with dopamine receptor antagonists in schizophrenia (Parkinson syndrome).

Currently, the principal symptomatic treatment for PD is based upon administration of dopamine-replacement therapy (e.g., levodopa (L-DOPA)) and/or therapy with dopamine receptor agonists (e.g., apomorphine). Long-term treatment with either therapy may lead to the development of dyskinesia, which is the most important motor complication that may arise in Parkinson patients. Although patients treated with dopamine receptor agonists are less prone to develop severe dyskinesia, other non-motor side effects such as somnolence, constipation, dizziness, nausea and hallucination have been reported to be increased with this treatment option. Concerning L-DOPA, long-term use of this treatment option leads to a reduction of its anti-parkinsonian efficacy and to the development of L-DOPA-induced dyskinesia (LID).

LID is characterised by a mixture of choreiform, dystonic or ballistic/myoclonic movements that are observed after L-DOPA administration. It is reported that about 30% of the Parkinson patients will experience dyskinesia after 4-6 years of treatment with L-DOPA while close to 90% will suffer from this complication after 9 years. Although the cause of dyskinesia remains unknown, the main risk factor for the development of LID is young age at PD onset, the disease severity and duration as well as a high initial dose of L-DOPA treatment. Ultimately, this complication severely impairs the quality of life and well-being of the patient and therefore limits the use of this drug as most important therapeutic agent.

According to the literature, LID may be related to the pulsatile and intermittent nature of L-DOPA therapy. Upon systemic administration of L-DOPA to Parkinson patients, dopamine is formed, stored and released by the remaining dopaminergic terminals as well as by other cellular components present in the striatum such as serotoninergic neurons. Since the striatal serotonin system remains relatively spared in most PD patients, it is believed to play an important role in determining the efficacy of L-DOPA therapy.

Many attempts have been made to develop serotonergic agents for the prevention or treatment of LID but these attempts have had limited success in patients. So far, the only LID treatment used clinically is the NMDA receptor antagonist Amantadine (Snow BJ, et al. The effect of amantadine on levodopa-induced dyskinesias in Parkinson's disease: a double-blind, placebo-controlled study. Clin Neuropharmacol 2000;23(2):82-5). A review of clinical studies indicated a short term improvement of dyskinesia, but the duration of the beneficial effect still remained unknown (Warren and Burn, Advances in clinical neurosciences and rehabilitation 4 (2004) 38-41). The problems associated with the use of Amantadine include confusion, hallucinations and withdrawal symptoms.

Based on the clinical results obtained with different 5-HT_{1A} agonists such as buspirone, sarizotan and tandospirone, it became evident that the 5-HT_{1A} component is not sufficient to treat the development of LID in Parkinson's patients in a way that makes a meaningful difference for patients. According to a recently published study, it is now suggested that the combination of a 5-HT_{1A} agonist with a 5-HT_{1B} agonist could prevent the dysregulated release of dopamine by serotonin neurons and therefore be beneficial for the treatment of LID (Carta M, Carlsson T, Kirik D and Björklund A. Dopamine released from 5-HT terminals is the cause of L-DOPA-induced dyskinesia in Parkinsonian rats, Brain 2007 130: 1819-1833). Although this combination has shown some positive results in the rat model of dyskinesia, the administration of Ru-24969, a substance acting through the joint stimulation of the 5-HT_{1A} and 5-HT_{1B} receptors, was associated with worsening of motor symptoms and/or development of side effects in monkey studies (Iravani MM, Jackson MJ, Kuoppamäki M, Smith LA, Jenner P. 3,4-methylenedioxymethamphetamine (ecstasy) inhibits dyskinesia expression and normalizes motor activity in 1-methyl-4-phenyl-1,2,3,6-tetrahydropyridine-treated primates, J Neurosci. 2003 23:9107-9115). These observations have prohibited its further development for the treatment of Parkinson patients with LID.

WO 2010/063486 discloses the use of Eltoprazine for the treatment of LID. The peculiarity of the invention underlying WO 2010/063486 compared to former treatment approaches for L-DOPA-induced dyskinesia was the therapeutic efficiency of Eltoprazine unknown in the prior art, which was based on a unique receptor profile with several components potentiating each other in an unexpected way. Prior to WO 2010/063486, the pharmacological action of Eltoprazine had mainly been attributed to its agonistic effect on the 5-HT_{1A} and 5-HT_{1B} receptors (see Schipper J, Tulp MTM, Sijbesma H. Neurochemical profile of Eltoprazine. Drug metabolism and Drug interactions 1990 8:85-114). In WO 2010/063486 it is described that Eltoprazine acts as an agonist at the 5-HT_{2C} receptor in human recombinant cell lines, in addition to its partial agonistic action on 5-HT_{2A} and 5-HT_{1B} receptors as well as its weak agonistic action on the 5-HT_{1A} receptor.

In light of the great need for efficacious treatment alternatives for LID patients and the limited progress that has been made so far, there is still a great need for pharmaceutical agents that are active in the treatment of disorders such as LID.

Thus, the present invention relates to the use of a 4-substituted 1-aryl-piperazine selected from OPC-14523 and OPC-34712 and their salts, which we have determined possess a unique receptor profile, which has so far not been identified in the prior art.

### SUMMARY OF THE INVENTION

The present invention relates to a novel method of treating or preventing L-DOPA-induced dyskinesia in a subject in need thereof.

Thus, in a first aspect, the present invention relates to a method of treatment or prevention of L-DOPA-induced dyskinesia in a subject in need thereof, comprising the step of administering a therapeutically effective amount of a 4-substituted 1-aryl-piperazine selected from OPC-14523 and OPC-34712, particularly OPC-14523, or a pharmaceutically acceptable salt thereof.

In a second aspect, the present invention relates to a composition comprising a therapeutically effective amount of a 4-substituted 1-aryl-piperazine selected from OPC-14523 and OPC-34712, particularly OPC-14523, or a pharmaceutically acceptable salt thereof for use in the treatment or prevention of L-DOPA-induced dyskinesia, particularly OPC-14523.

In particular embodiments, the pharmaceutically acceptable salt is selected from a monohydrochloride; and a monomethanesulfonate, particularly a monohydrochloride.

In particular embodiments, OPC-14523 or OPC-34712 is for administration in a range from about 5 mg to about 75 mg/day, or in a range from about 5 mg to about 60 mg/day, or in a range from about 5 mg to about 50 mg/day, or in a range from about 5 mg to about 40 mg/day, or in a range from about 5 mg to about 20 mg/day, or in a range from about 5 mg to about 15 mg/day.

In particular such embodiments, OPC-14523 or OPC-34712 is for administration in a range from about 10 mg to about 15 mg/day.

In particular embodiments, OPC-14523 or OPC-34712 is for administration at about 5 mg/day, or OPC-14523 or OPC-34712 is for administration at about 10 mg/day, or OPC-14523 or OPC-34712 is for administration at about 15 mg/day, or OPC-14523 or OPC-34712 is for administration at about 20 mg/day, or OPC-14523 or OPC-34712 is for administration at about 40 mg/day, or OPC-14523 or OPC-34712 is for administration at about 60 mg/day.

In particular embodiments, OPC-14523 or OPC-34712 or a pharmaceutically acceptable salt thereof is for administration once a day, twice a day (b.i.d.), or three times a day.

In particular such embodiments, OPC-14523 or OPC-34712 or a pharmaceutically acceptable salt thereof is for administration twice a day (b.i.d.).

In particular such embodiments, the daily dosage for said administration twice a day (b.i.d.) is split into a first dose of about 55 to 65% of the total daily dosage amount, and a second dose comprising the remaining total daily dosage amount.

In particular such embodiments, the second dosage is administered at about lunchtime.

In particular embodiments, the subject to be treated suffers from a movement disorder.

In particular such embodiments, the movement disorder is selected from parkinsonism, restless legs syndrome (RLS), and Chorea Huntington, particularly parkinsonism.

In particular such embodiments, the parkinsonism is idiopathic (Parkinson's disease; PD).

In particular embodiments, the subject is undergoing long-term treatment with L-DOPA therapy indicated for the treatment of PD.

In particular such embodiments, the subject is undergoing treatment with dopamine receptor agonists.

In particular embodiments, said treatment or prevention comprises the additional administration with at least one additional pharmaceutical agent.

In particular such embodiments, the additional pharmaceutical agent(s) is/are selected from the group of (i) an agent which has been shown to be effective for the treatment of PD, and (ii) a decarboxylase inhibitor, particularly selected from benserazide and carbidopa.

In particular embodiments, OPC-14523 or OPC-34712 or a pharmaceutically acceptable salt thereof is in the form of an oral formulation.

In particular embodiments, said therapeutically effective amount of OPC-14523 or OPC-34712 or a pharmaceutically acceptable salt thereof is administered at least about 10 minutes before the administration of a therapeutically effective amount of L-DOPA.

In particular embodiments, said method or composition is for the prevention of L-DOPA-induced dyskinesia, wherein OPC-14523 or OPC-34712 or a pharmaceutically acceptable salt thereof is administered to a subject receiving L-DOPA, wherein said subject is still free of symptoms of L-DOPA-induced dyskinesia, and the treatment with said composition is started at the latest 9 years after the beginning of said L-DOPA treatment.

In particular embodiments, said subject receives L-DOPA as first-line treatment of Parkinson's disease.

In particular such embodiments, said subject receives L-DOPA either in combination with a dopamine agonist or as a second-line treatment of Parkinson's disease.

In particular embodiments, said treatment comprises administration of an increased dosage of L-DOPA compared to the dosage used by said subject without the administration of OPC-14523 or OPC-34712.

In particular such embodiments, said therapeutically effective amount of OPC-14523 or OPC-34712 is selected such that the L-DOPA-induced dyskinesia caused by said increased dosage of L-DOPA is kept at, or below, the level which was present in said subject without the administration of said therapeutically effective amount of OPC-14523 or OPC-34712.

### BRIEF DESCRIPTION OF THE DRAWINGS

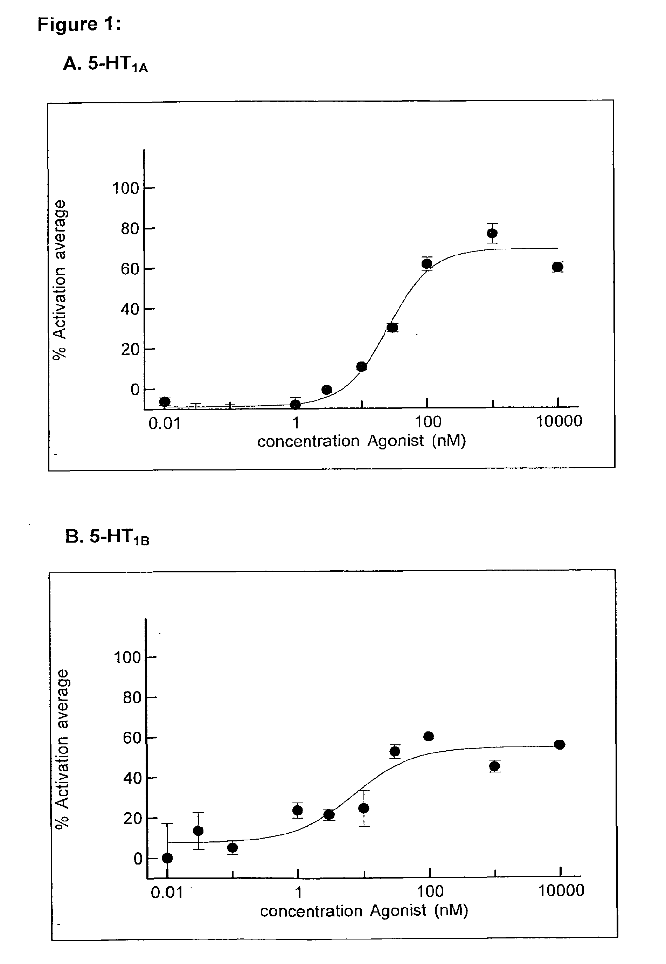

**Figure 1** shows the results of functional assays of OPC-14523 at target receptors 5-HT_{1A} and 5-HT_{1B}.

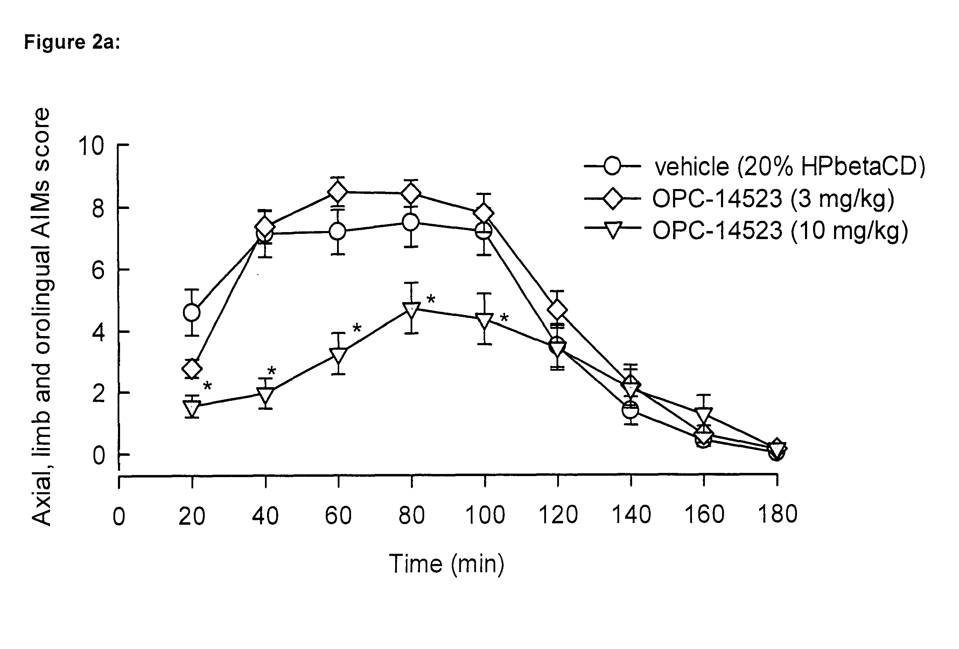

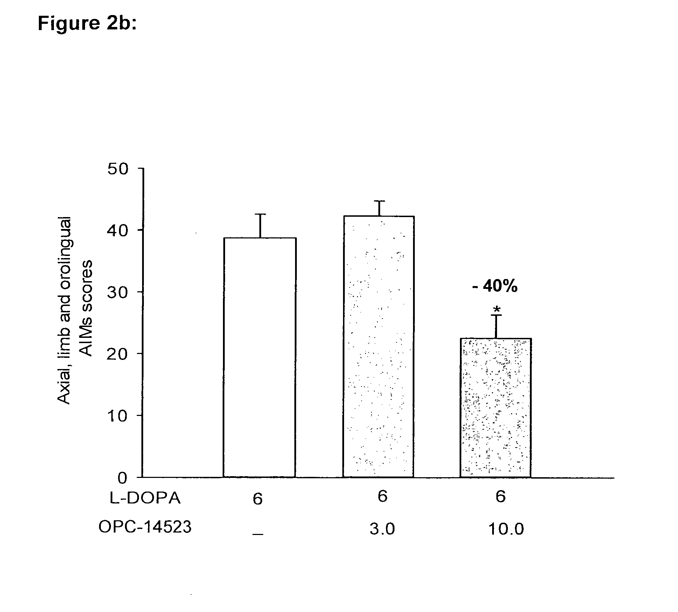

**Figure 2** shows the effect of OPC-14523 on L-DOPA-induced dyskinesia (LID) in an *in vivo* model: **Figure 2a** shows the effect of OPC-14523 on the time course of L-DOPA-induced dyskinesia ; **Figure 2b** shows the effect of OPC-14523 on the cumulative AIMs score of L-DOPA-induced dyskinesia over the total measurement period of 180 minutes ; OPC-14523 was dissolved in 20% HPBCD/distilled water and injected p.o. 10 min before L-DOPA; L-DOPA/benserazide (6/15 mg/kg) was dissolved in saline and injected i.p. 20 min before the test.

### DETAILED DESCRIPTION OF THE INVENTION

In a first aspect, the present invention relates to a method of treatment or prevention of L-DOPA-induced dyskinesia in a subject in need thereof, comprising the step of administering a therapeutically effective amount of a 4-substituted 1-aryl-piperazine selected from OPC-14523 and OPC-34712, particularly OPC-14523, or a pharmaceutically acceptable salt thereof.

In a second aspect, the present invention relates to a composition comprising a therapeutically effective amount of a 4-substituted 1-aryl-piperazine selected from OPC-14523 and OPC-34712, particularly OPC-14523, or a pharmaceutically acceptable salt thereof for use in the treatment or prevention of L-DOPA-induced dyskinesia.

OPC-14523 (1-[3-[4-(3-Chlorophenyl)piperazin-1-yl]propyl]-5-methoxy-1,2,3,4-tetrahydroquinolin-2-one; structural formula see below) is also known by its code VPI-013 (Oshiro et al., 3,4-dihydro-2(1 H)-quinolinone as a novel antidepressant drug: synthesis and pharmacology of 1-[3-[4-(3-chlorophenyl)-1-piperazinyl]propyl]-3,4-dihydro-5-methoxy-2(1H)-quinolinone and its derivatives. J Med Chem. 43 (2000) 177-89). The compound was originally developed by the Japanese company Otsuka Pharmaceutical Co., Ltd. for the treatment of major depressive disorder, neuropathic pain and for both male and female sexual dysfunction. The highest clinical development stage the compound has reached is Phase 2 for major depressive disorder, neuropathic pain and female sexual dysfunction. Currently the clinical development of the compound has been suspended for all indications. In the Phase 1 studies of the compound, no adverse events had been reported. In 2004, the compound was licensed to Vela Pharmaceuticals (acquired by Pharmas Corp. in 2006) for development and marketing in the United States and Europe.

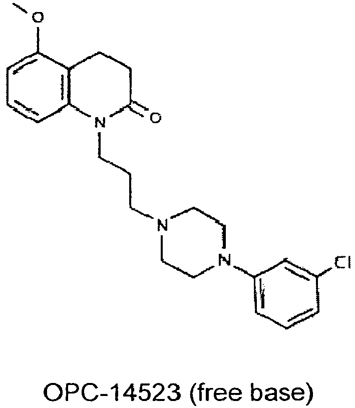

Known sub-micromolar molecular targets of OPC-14523 included 5-HT_{1A} (5HT (serotonin) receptor 1A) (agonist), sigma 1 and 2 receptors (agonist) and serotonin transporter (inhibitor). Further targets with micromolar potency are the dopamine and the norepinephrine transporters. Additionally the compound has been tested against the following targets but found to be inactive: MAO-A, MAO-B, Muscarinic acetylcholine receptors M1, M2 and M3 and the NMDA receptor (Tottori et al. Antidepressant-like responses to the combined sigma and 5-HT1A receptor agonist OPC-14523. Neuropharmacology. 41 (2001) 976-88).

Brexpiprazole (7-{4-[4-(1-benzothiophen-4-yl)piperazin-1-yl]butoxy}quinolin-2(1 H)-one; structural formula see below) is also known by its research code OPC-34712 (WO 2006/112464: Piperazine-substituted benzothiophenes and their preparation, pharmaceutical compositions and use in the treatment of mental disorders). The compound was originally developed by the Japanese company Otsuka Pharmaceutical Co., Ltd. for the treatment of mental disorders. Clinical studies have been performed, or are still on-going, *inter alia* in psychosis (Phase 3, on-going), depression (Phase 3, on-going), schizophrenia, and ADHD (Phase 2, completed 2011). In 2011, the compound was partnered with Lundbeck.

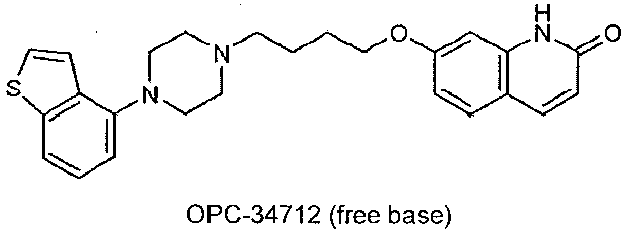

Known molecular targets of OPC-34712 included 5-HT_{1A} (5HT (serotonin) receptor 1A) (partial agonist), 5-HT₇ (antagonist), 5-HT_{2A} (antagonist), adrenalin α1 receptor (antagonist), and D2 dopamine receptor (partial agonist). Furthermore, the compound was reported to have a serotonin uptake/reuptake inhibitory effect.

The peculiarity of this invention compared to former treatment approaches for L-DOPA-induced dyskinesia is the so far unknown therapeutic efficiency of 4-substituted 1-aryl-piperazine selected from OPC-14523 and OPC-34712, which is based on a unique receptor profile with several components potentiating each other in an unexpected way. Although the pharmacological action of OPC-14523 and OPC-34712 was mainly attributed to their agonistic effect on the 5-HT_{1A} receptor (see above), we have observed that OPC-14523 acts as well as a partial agonist at the 5-HT_{1B} receptor in human recombinant cell lines (see Figure 1). The same observation can be made with OPC-34712.

Furthermore, it has surprisingly been observed that 4-substituted 1-arylpiperazines such as OPC-14523 act as well as antagonists on the 5-HT_{2B} receptor. 5-HT_{2B} receptors are involved in various functions of the CNS and cardiovascular system. Prolonged systemic exposure to 5-HT_{2B} agonists is considered to be the underlying cause of drug-induced valvular heart disease (VHD; Cosyns B Drug-induced valvular heart disease. Heart, 99(1):7-12, 2013). In this pathology one or more heart valves don't function properly and blood pumping of the heart is compromised leading to regurgitation (backward leakage of blood) and finally heart failure, depending on the severity and duration of valve insufficiency. Treatment options for VHD are limited, mainly consisting of cardiac surgery, i.e. valve replacement.

VHD has been observed in patients treated with the weight-loss drugs fenfluramine and dexfenfluramine. Due to cardiac side effect these drugs were removed from the U.S. market in 1997 (Rothman & Baumann, Expert Opin Drug Saf. 2009 May;8(3):317-29). Dopamine agonists of the ergotamine type like pergolide and cabergoline were identified as 5-HT2b receptor agonists and their valvulopathic activity is reflected by the higher incidence of VHD in patients treated with these compounds (Zanettini R et al. Valvular Heart Disease and the Use of Dopamine Agonists for Parkinson's Disease. N Engl J Med 2007;356:39-46): Pergolide was removed from the US market in 2007 (Elangbam C Drug-induced Valvulopathy: An Update. Toxicologic Pathology, 38: 837-848, 2010).

Currently several strategies have been suggested to evaluate the agonistic potential of 5-HT_{2B} ligands (Huang X-P et al. Parallel Functional Activity Profiling Reveals Valvulopathogens Are Potent 5-Hydroxytryptamine 2B Receptor Agonists: Implications for Drug Safety Assessment. Mol Pharmacol 76:710-722, 2009; Setola V et al. 3,4-Methylenedioxymethamphetamine (MDMA, "Ecstasy") Induces Fenfluramine-Like Proliferative Actions on Human Cardiac Valvular Interstitial Cells in Vitro. Mol Pharmacol 63:1223-1229, 2003) in vitro. However, the validation of these models is difficult, because no accepted animal model for drug-induced VHD is available (Elangbam, loc. cit.). Due to the severity of this condition and the lack of reliable preclinical models the FDA recommends the inclusion of serial echocardiography measurements in the clinical development of weight loss drugs, which often target 5-HT2 receptors (FDA Guidance for Industry: Developing Products for Weight Management, DRAFT GUIDANCE, February 2007).

Compounds with 5-HT_{2B} agonistic activity thus have a high risk to induce VHD in patients which will usually be observed not before large late stage clinical trials or after market authorization. The only way to avoid this liability is developing compounds without agonistic activity at the 5-HT_{2B}.

Thus, there is still a great need for pharmaceutical agents that are active in the treatment of disorders such as LID by having an agonistic effect on the 5-HT_{1A} and 5-HT_{1B} receptors, while simultaneously not having an agonistic effect on the 5-HT_{2B} receptor.

As used herein, the term "subject" encompasses mammals including animals and humans.

The term "agonist" refers to a substance that binds to a receptor and mimics the cellular effect of the native or endogenous ligand for the same receptor. The term agonist includes the class of agents called full agonists, which bind and display full efficacy at the receptor, and partial agonists, which have only partial efficacy at the receptor. Partial agonists may also be seen as competitive antagonists, competing away the endogenous ligand when it is in excess or give a sub maximal response when inadequate amount of endogenous ligand is present. The term "activation" refers to the state of a receptor when an agonist is bound to it.

The term "dyskinesia" refers to involuntary movements similar to a tic or chorea, which interferes with the performance of voluntary movements. The term dyskinesia may be used in relation to Parkinson's disease and/or other extrapyramidal disorders. In the case of Parkinson's disease, dyskinesia may occur as a complication of dopaminomimetic therapy with L-DOPA (LID), or, in rare cases, after treatment with dopamine receptor agonists such as apomorphine. In LID, dyskinesia most commonly occurs in two forms: peak dose, also known as improvement-dyskinesia-improvement (IDI), which most commonly occur at the time of peak L-DOPA plasma concentrations, and diphasic dyskinesia also known as dyskinesia-improvement-dyskinesia (DID), which occur when the L-DOPA concentration rises or falls.

The term "levodopa" or "L-DOPA" refers to a therapeutic strategy use to treat PD. L-DOPA is defined as a dopamine precursor because L-DOPA needs to be decarboxylated to dopamine to be effective. On the other hand, the term "dopamine receptor agonist" refers to dopamine D1, D2 or D3 receptor agonists, which bind directly to their respective receptors to induce an effect.

The term "treat" is used herein to mean to relieve or alleviate at least one symptom of a disease in a subject. Within the meaning of the present invention, the term "treat" also denotes to arrest and/or reduce the risk of worsening a disease.

The term "prevent" is used herein to mean to prevent, or to delay the onset of, the manifestation at least one symptom of a disease in a subject. Within the meaning of the present invention, the term "prevent" accordingly denotes to delay the onset (*i.e.,* the period prior to clinical manifestation of a disease) and/or reduce the risk of developing a disease.

The term "therapeutically effective" applied to dose or amount refers to that quantity of a compound or pharmaceutical composition sufficient to result in a desired activity upon administration to a mammal in need thereof.

The phrase "pharmaceutically acceptable", as used in connection with compositions of the invention, refers to molecular entities and other ingredients of such compositions that are physiologically tolerable and do not typically produce untoward reactions when administered to a mammal (e.g., human). The term "pharmaceutically acceptable" may also mean approved by a regulatory agency of the Federal or a state government or listed in the U.S. Pharmacopeia or other generally recognized pharmacopeia for use in mammals, and more particularly in humans.

The term "salt" is defined as a chemical containing different charged components. The term salt also includes hydrates and solvates. Contemplated in the instant description are pharmaceutically acceptable salts, which salts may include, but are not limited to, acid addition salts, such as those made with hydrochloric, sulphuric, nitric, phosphoric, acetic, maleic, fumaric, tartaric, citric, benzoic, methane sulphonic, naphthalene sulphonic, p-toluene sulphonic acid. All of these salts (or other similar salts) may be prepared by conventional means. The nature of the salt is not critical, provided that it is non-toxic and does not substantially interfere with the desired pharmacological activity.

OPC-14523 or OPC-34712 may be used according to the invention as their free base or in the form of any pharmaceutically acceptable salt thereof, and solvates. Any references to OPC-14523 or OPC-34712 in this description should be understood as also referring to such free forms, salts and solvates.

In particular embodiments, the pharmaceutically acceptable salt is selected from a monohydrochloride; and a monomethanesulfonate, particularly a monohydrochloride.

In conjunction with the methods of the present invention, also provided are pharmaceutical compositions for use in such methods comprising a therapeutically effective amount of OPC-14523 or OPC-34712. The compositions of the invention may further comprise a carrier or excipient (all pharmaceutically acceptable). The compositions may be formulated e.g. for once-a-day administration, twice-a-day administration, three times a day administration, or as a modified release formulation.

The term "carrier" applied to pharmaceutical compositions of the invention refers to a diluent, excipient, or vehicle with which an active compound (e.g., OPC-14523) is administered. Such pharmaceutical carriers may be sterile liquids, such as water, saline solutions, aqueous dextrose solutions, aqueous glycerol solutions, and oils, including those of petroleum, animal, vegetable or synthetic origin, such as peanut oil, soybean oil, mineral oil, sesame oil and the like. Suitable pharmaceutical carriers are described in "Remington's Pharmaceutical Sciences" by A.R. Gennaro, 20th Edition.

In particular embodiments, OPC-14523 or OPC-34712 is for administration in a range from about 5 mg to about 75 mg/day, or in a range from about 5 mg to about 60 mg/day, or in a range from about 5 mg to about 50 mg/day, or in a range from about 5 mg to about 40 mg/day, or in a range from about 5 mg to about 20 mg/day, or in a range from about 5 mg to about 15 mg/day.

In particular such embodiments, OPC-14523 or OPC-34712 is for administration in a range from about 10 mg to about 15 mg/day.

In particular embodiments, OPC-14523 or OPC-34712 is for administration at about 5 mg/day, or OPC-14523 or OPC-34712 is for administration at about 10 mg/day, or OPC-14523 or OPC-34712 is for administration at about 15 mg/day, or OPC-14523 or OPC-34712 is for administration at about 20 mg/day, or OPC-14523 or OPC-34712 is for administration at about 40 mg/day, or OPC-14523 or OPC-34712 is for administration at about 60 mg/day.

The active ingredient (e.g., OPC-14523 or OPC-34712) or the composition of the present invention may be used for the treatment of at least one of the mentioned disorders, wherein the treatment is adapted to or appropriately prepared for a specific administration as disclosed herein (e.g., to once-a-day, twice-a-day, or three times a day administration). For this purpose the package leaflet and/or the patient information contains corresponding information.

In particular embodiments, OPC-14523 or OPC-34712 or a pharmaceutically acceptable salt thereof is for administration once a day, twice a day (b.i.d.), three times a day, or for administration as a modified release formulation.

In particular such embodiments, OPC-14523 or OPC-34712 or a pharmaceutically acceptable salt thereof is for administration twice a day (b.i.d.).

In particular such embodiments, the daily dosage for said administration twice a day (b.i.d.) is split into a first dose of about 55 to 65% of the total daily dosage amount, and a second dose comprising the remaining total daily dosage amount.

In particular such embodiments, the second dosage is administered at about lunchtime.

In particular embodiments, the subject to be treated is not treated with an additional pharmaceutical agent(s) selected from the group of (i) a COX-2 inhibitor, particularly a COX-2 inhibitor listed in WO 2004/045509 or in WO 2005/048999; (ii) Pipamperone; (iii) an AMPA receptor antagonist, particularly an AMPA receptor antagonist listed in WO 2009/011412; and (iv) Flibanserin.

In particular embodiments, the subject to be treated suffers from a movement disorder.

In particular such embodiments, the movement disorder is selected from parkinsonism, restless legs syndrome (RLS), and Chorea Huntington, particularly parkinsonism.

In particular such embodiments, the parkinsonism is idiopathic (Parkinson's disease, PD).

In particular embodiments, the subject is undergoing long-term treatment with L-DOPA therapy indicated for the treatment of PD.

In particular such embodiments, the subject is undergoing treatment with dopamine receptor agonists.

In particular embodiments, said treatment or prevention comprises the additional administration with at least one additional pharmaceutical agent.

In particular such embodiments, the additional pharmaceutical agent(s) is/are selected from the group of (i) an agent which has been shown to be effective for the treatment of PD, and (ii) a decarboxylase inhibitor, particularly selected from benserazide and carbidopa. In particular such embodiments, the additional pharmaceutical agent(s) is/are not an agent selected from the group of (i) a COX-2 inhibitor, particularly a COX-2 inhibitor listed in WO 2004/045509 or in WO 2005/048999; (ii) Pipamperone; (iii) an AMPA receptor antagonist, particularly an AMPA receptor antagonist listed in WO 2009/011412; and (iv) Flibanserin.

In one embodiment, a therapeutically effective amount of OPC-14523 or OPC-34712 is administered before the administration of a therapeutically effective amount of L-DOPA, for example at least about 10 15, 20, 25, 30, 35, or 40 minutes before the administration of a therapeutically effective amount of L-DOPA. In particular embodiments, a therapeutically effective amount of OPC-14523 or OPC-34712 is administered between about 10 and 25 minutes before the administration of a therapeutically effective amount of L-DOPA. This administration scheme should allow synchronizing the peak plasma level of L-DOPA with OPC-14523 or OPC-34712 plasma levels, due to the faster uptake of L-DOPA in comparison to OPC-14523 or OPC-34712. In doing so, the brain-activity of both substances should occur during the same time period.

In particular embodiments, said therapeutically effective amount of OPC-14523 or OPC-34712 or a pharmaceutically acceptable salt thereof is administered at least about 10 minutes before the administration of a therapeutically effective amount of L-DOPA.

The term "about" or "approximately" usually means within 20%, alternatively within 10%, including within 5% of a given value or range. Alternatively, especially in biological systems, the term "about" means within about a log (*i.e.,* an order of magnitude), including within a factor of two of a given value.

In another embodiment, a therapeutically effective amount of OPC-14523 or OPC-34712 is administered simultaneously with the administration of a therapeutically effective amount of L-DOPA, for example by administration of OPC-14523 or OPC-34712 and L-DOPA within about 2 minutes, within about 1 minute, or as part of a combination product. This administration scheme should allow a simplified means of administration for patients handicapped by the underlying disease or disorder.

In another embodiment, a therapeutically effective amount of OPC-14523 or OPC-34712 is administered twice per day. In particular, OPC-14523 or OPC-34712 is administered once in the morning, particularly once prior to the first L-DOPA administration, and once in the middle of the day, particularly at about lunchtime, wherein the lunchtime treatment is between about 6 and 10, particularly between about 7 and 9 hours before the patient wishes to go to bed, or, alternatively, prior to the mid-point of daily L-DOPA dosages (e.g. prior to the third L-DOPA dosage in a five-time-daily L-DOPA dosage regimen, or between the third and fourth L-DOPA dosages in a six-time-daily L-DOPA dosage regimen). Such an administration should allow for the OPC-14523 or OPC-34712 plasma level to ebb away during evening and at nighttime, reducing the risk of an impaired REM-*(rapid eye* movement)-activity.

In certain such embodiments, the treatment is the treatment of diphasic dyskinesia (DID).

In another embodiment, the first dosage of OPC-14523 or OPC-34712 consists of about 55 to 65% of the total daily dosage amount, and the second dose of OPC-14523 or OPC-34712 comprises the remaining total daily dosage amount.

The active ingredient (e.g., OPC-14523 or OPC-34712) or the composition of the present invention may be used for the manufacture of a medicament for the treatment of at least one of the mentioned disorders, wherein the medicament is adapted to or appropriately prepared for a specific administration as disclosed herein (e.g., to once-a-day, twice-a-day, or three times a day administration). For this purpose the package leaflet and/or the patient information contains corresponding information.

According to the present invention, the dosage form of OPC-14523 or OPC-34712 may be a solid, semisolid, or liquid formulation according to the following.

OPC-14523 or OPC-34712 may be administered orally, topically, parenterally (e.g. intravenous, subcutaneous), or mucosally (e.g., buccally, by inhalation, or rectally) in dosage unit formulations containing conventional non-toxic pharmaceutically acceptable carriers. OPC-14523 or OPC-34712 may be administered orally in the form of a capsule, a tablet, granules, pellets or the like, or as a semi-solid, or liquid formulation (see Remington's Pharmaceutical Sciences, 20th Edition, by A.R. Gennaro).

In particular embodiments, the free base of OPC-14523 or OPC-34712 or a pharmaceutically acceptable salt thereof is in the form of an oral formulation.

In one embodiment of the invention, OPC-14523 or OPC-34712 is administered in a modified release formulation. Modified release dosage forms provide a means for improving patient compliance and for ensuring effective and safe therapy by reducing the incidence of adverse drug reactions. Compared to immediate release dosage forms, modified release dosage forms may be used to prolong pharmacologic action after administration, and to reduce variability in the plasma concentration of a drug throughout the dosage interval, thereby eliminating or reducing sharp peaks.

A modified release dosage form may comprise a core either coated with or containing a drug. The core is then coated with a release-modifying polymer within which the drug is dispersed. The release-modifying polymer disintegrates gradually, releasing the drug over time. Thus, the outer-most layer of the composition effectively slows down and thereby regulates the diffusion of the drug across the coating layer when the composition is exposed to an aqueous environment, i.e. the gastrointestinal tract. The net rate of diffusion of the drug is mainly dependent on the ability of the gastric fluid to penetrate the coating layer or matrix and on the solubility of the drug itself.

The formulations of the invention may be delivered parenterally, i.e., by intravenous (i.v.), intracerebroventricular (i.c.v.), subcutaneous (s.c.), intraperitoneal (i.p.), intramuscular (i.m.), subdermal (s.d.), or intradermal (i.d.) administration, by direct injection, via, for example, bolus injection or continuous infusion, or as a patch formulation for dermal application. Formulations for injection may be presented in unit dosage form, e.g., in ampoules or in multi-dose containers, with an added preservative. Alternatively, the active ingredient may be in powder form for reconstitution with a suitable vehicle, e.g., sterile pyrogen-free water, before use.

The optimal therapeutically effective amount may be determined experimentally, taking into consideration the exact mode of administration, form in which the drug is administered, the indication toward which the administration is directed, the subject involved (e.g., body weight, health, age, sex, etc.), and the preference and experience of the physician or veterinarian in charge.

In certain embodiments, the invention relates to the use of OPC-14523 or OPC-34712 in the preventive treatment of a patient receiving L-DOPA treatment, i.e. where the L-DOPA-treated patient is still free of symptoms.

Thus, in particular embodiments, said method or composition is for the prevention of L-DOPA-induced dyskinesia, wherein OPC-14523 or OPC-34712 or a pharmaceutically acceptable salt thereof is administered to a subject receiving L-DOPA, wherein said subject is still free of symptoms of L-DOPA-induced dyskinesia, and the treatment with said composition is started at the latest 9 years after the beginning of said L-DOPA treatment.

In one such embodiment, OPC-14523 or OPC-34712 is for administration to a patient, who receives L-DOPA as first-line treatment of PD, particularly within about 9, about 6, about 4, about 3 years or about 2 years from start of L-DOPA treatment. In a particular embodiment, the patient is at the age of at least 60, 65 or 70 years.

In particular such embodiments, said subject receives L-DOPA either in combination with a dopamine agonist or as a second-line treatment of Parkinson's disease.

In particular such embodiment, OPC-14523 or OPC-34712 is for administration to a patient, who either receives L-DOPA in combination with one or more dopamine receptor agonists, or as second-line treatment of PD after prior treatment with one or more dopamine receptor agonists, particularly within about 9, about 6, about 4, about 3 years or about 2 years from start of L-DOPA treatment. Alternatively, the patient is treated with OPC-14523 or OPC-34712 from the point in time on, where such L-DOPA combination or second-line treatment is started.

In particular embodiments, said treatment comprises administration of an increased dosage of L-DOPA compared to the dosage used by said subject without the administration of OPC-14523 or OPC-34712.

Sometimes a patient is more interested in reducing the periods of insufficient motility ("off-phase") than in the reduction of dyskinesia ("on-phase"). The dosage of a treatment with L-DOPA and/or dopamine agonists, however, can only be increased as long as the occurrence of LID is still bearable. Therefore, in another embodiment, OPC-14523 or OPC-34712 is used to keep the LID symptoms at a stable level (rather than reducing them), thereby enabling to increase the dosage of a treatment with L-DOPA and/or dopamine agonists and as such decreasing the off-phase. Thus, this embodiment relates to a method of using OPC-14523 or OPC-34712 or a pharmaceutically acceptable salt thereof for, or the use of OPC-14523 or OPC-34712 or a pharmaceutically acceptable salt thereof for the manufacture of a medicament for, maintaining the level of L-DOPA-induced dyskinesia in a patient that is treated with an increased amount of L-DOPA and/or dopamine receptor agonists. In the context of the present invention, the term "maintaining the level" has the meaning that dyskinetic events are kept at a level that shows about the same grade and/or frequency of dyskinetic events as at beginning of OPC-14523 or OPC-34712 treatment, and the term "increased amount of L-DOPA and/or dopamine receptor agonists" has the meaning that the patient is treated with a higher amount of L-DOPA when compared with the amount at the beginning of OPC-14523 or OPC-34712 treatment.

In particular such embodiments, said therapeutically effective amount of OPC-14523 or OPC-34712 is selected such that the L-DOPA-induced dyskinesia caused by said increased dosage of L-DOPA is kept at, or below, the level which was present in said subject without the administration of said therapeutically effective amount of OPC-14523 or OPC-34712.

Thus, the invention relates to a method/composition/use, wherein
a) an effective amount of OPC-14523 or OPC-34712 or a pharmaceutically acceptable salt thereof is administered to a subject;
b) such effective amount is selected to that effect that the L-DOPA induced dyskinesia is kept at a level which was present in the subject without the administration of such effective amount; and
c) the dosage of L-DOPA is increased.

Dosage units for rectal application may be solutions or suspensions or may be prepared in the form of suppositories or retention enemas comprising OPC-14523 or OPC-34712 in a mixture with a neutral fatty base, or gelatin rectal capsules comprising the active substances in admixture with vegetable oil or paraffin oil.

Toxicity and therapeutic efficacy of the compositions of the invention may be determined by standard pharmaceutical procedures in experimental animals, e.g., by determining the LD₅₀ (the dose lethal to 50% of the population) and the ED₅₀ (the dose therapeutically effective in 50% of the population). The dose ratio between therapeutic and toxic effects is the therapeutic index and it may be expressed as the ratio LD₅₀/ED₅₀. Compositions that exhibit large therapeutic indices are preferred.

Suitable daily doses of the active ingredient of the invention (OPC-14523 or OPC-34712) in therapeutic treatment of humans are within the range from about 5 mg to about 75 mg, or from about 5 mg to about 60 mg, or from about 5 mg to about 50 mg, or from about 10 mg to about 20 mg, or from about 10 mg to about 15 mg, such as 10mg or 15 mg or 20 mg or 30 mg or 40 mg or 60mg or 80mg, per day. For example the daily dose may be body weight-adjusted such as 40 mg/day up to 80 kg body weight or 60 mg/day for patients with a body weight of ≥ 80 kg. In modified release formulations the amounts of active ingredient per day could also be higher due to reduced bioavailablitity, e.g. up to 100 mg/day. An equimolar amount of another pharmaceutically acceptable salt, a solvate, a conjugate or a derivative thereof, such as OPC-14523 or OPC-34712 hydrochloride, is also suitable. For pediatric subjects aged 4-14, OPC-14523 or OPC-34712 may be administered as an oral, liquid dosage form, at reduced amounts, for example from about 1 mg/day, up to about 5 mg/day.

In particular embodiments, the daily dosage of OPC-14523 or OPC-34712 is between about 10 and 20 mg/day.

The daily doses indicated herein may be administered, for example, as one or two dosing units once, twice or three times per day. Suitable doses per dosage unit may therefore be the daily dose divided (for example, equally) between the number of dosage units administered per day, and will thus typically be about equal to the daily dose or one half, one third, one quarter or one sixth thereof. Dosages per dosage unit may thus be calculated from each daily dosage indicated herein. A daily dose of 5 mg, for example may be seen as providing a dose per dosage unit of, for example, about 5 mg, 2.5 mg, 1.67 mg, 1.25 mg and 0.83 mg, depending upon the dosing regimen chosen. Correspondingly, a dosage of 50 mg per day corresponds to dosages per dosing unit of, for example, about 50 mg, 25 mg, 16.7 mg, 12.5 mg, and 8.33 mg for corresponding dosing regimens. In other embodiments, especially in cases where only two daily doses are administered, an unequal split of the first and the second dosage is envisaged. In such cases, for example, the first dosage comprises about 55 to 65% of the total daily dosage. For example, a daily dosage of 10 mg is split into a first dosage of 6 mg and a second dosage of 4 mg. In cases, in which more than two administrations per day are envisaged, the dosage might be split also unequally, wherein the second and further dosages are reduced in comparison to the dosage before. For example, if three dosages per day are administered, the first dosage could be about one half of the total daily dosage, i.e. between about 40% to 60%, the second dosage could be about one third of the total daily dosage, i.e. between about 20% to 40%, and the third dosage could be about one sixth of the total daily dosage, i.e. between about 5% to 20%. For example, a daily dosage of 10 mg could be split into a first dosage of 6 mg, a second dosage of 3 mg and a third dosage of 1 mg.

In certain such embodiments, the treatment is the treatment of diphasic dyskinesia (DID).

Treatment duration may be short-term, e.g., several weeks (for example 8-14 weeks), or long-term until the attending physician deems further administration no longer is necessary.

OPC-14523 or OPC-34712 may be administered as a single antidyskinetic agent in combination with dopamine replacement therapy (e.g. L-DOPA) and/or therapy with dopamine receptor agonists (e.g. apomorphine) for the treatment of L-DOPA-induced dyskinesias. In particular embodiments, L-DOPA is administered together with a decarboxylase inhibitor, including but not limited to benserazide (e.g. in Levodopa comp. B STADA^{®}, Levopar^{®} Madopar^{®}, PK-Levo^{®}, or Restex^{®}), or carbidopa (e.g. in Nacom®, Dopadura C^{®}, Levobeta C^{®}, or Stalevol^{®}).

The term "combination" applied to active ingredients is used herein to define two separate pharmaceutical compositions, each comprising an active agent (e.g. OPC-14523 or OPC-34712, and another pharmaceutical composition comprising another agent prescribed for the treatment of motor disorders such as Parkinson disease), to be administered conjointly.

Within the meaning of the present invention, the term "conjoint administration" is used to refer to administration of OPC-14523 or OPC-34712, and a second active agent simultaneously in different compositions, or sequentially. For the sequential administration to be considered "conjoint", however, OPC-14523 or OPC-34712, and the second active agent must be administered separated by a time interval, which still permits the resultant beneficial effect for treating L-DOPA-induced dyskinesias in a mammal.

Another aspect of the present invention relates to OPC-14523 or OPC-34712, or of a pharmaceutically acceptable salt thereof, OPC-14523for use in the treatment of a disease selected from the group of: ADHD, weight disorders, other (non-LID) dyskinesias, Huntington's disease and restless leg syndrome.

Thus, in a particular embodiment the present invention relates to a method for administering an effective amount of OPC-14523 or OPC-34712 or a pharmaceutically acceptable salt thereof for improving the primary symptoms of PD, such as bradykinesia and/or OFF time, in a subject having Parkinson's disease, particularly to a subject suffering from L-DOPA induced dyskinesia, and to compositions comprising OPC-14523 or OPC-34712 or a pharmaceutically acceptable salt thereof for use in such methods.

Furthermore, in another aspect the present invention relates to a method for administering an effective amount of OPC-14523 or OPC-34712 or a pharmaceutically acceptable salt thereofOPC-14523, for improving pain in a subject having Parkinson's disease, particularly to a subject suffering from pain as a consequence of dystonic dyskinesia as well as of muscular rigidity, and to compositions comprising OPC-14523 or OPC-34712 or a pharmaceutically acceptable salt thereofOPC-14523 for use in such methods.

In addition to the strong antidyskinetic effect, OPC-14523 or OPC-34712:
- improves the primary symptoms of PD (bradykinesia, OFF time etc.),
- improves non-motor symptoms, e.g. depression, anxiety, and cognitive deficits, which are among of the most challenging co-morbidities in Parkinson's disease;
- reduces pain, which is an extremely inconvenient consequence of dystonic dyskinesia as well as of muscular rigidity;
- improves "on / off phenomenon";
- improves dystonic symptoms of PD; and
- improves Quality of Life in PD.

Thus, in a particular embodiment the present invention relates to a method for administering an effective amount of OPC-14523 or OPC-34712 or a pharmaceutically acceptable salt thereof for improving the primary symptoms of PD, such as bradykinesia and/or OFF time, in a subject having Parkinson's disease, particularly to a subject suffering from L-DOPA induced dyskinesia, and to compositions comprising OPC-14523 or OPC-34712 or a pharmaceutically acceptable salt thereof for use in such methods.

OPC-14523 or OPC-34712 may be used for the treatment of at least one of the mentioned disorders, wherein the treatment is adapted to or appropriately prepared for a specific administration as disclosed herein (e.g., to once-a-day, twice-a-day, or three times a day administration). For this purpose the package leaflet and/or the patient information contains corresponding information.

### EXAMPLES

The following examples illustrate the invention without limiting its scope.

### EXAMPLE 1: In vitro 5-HT Activity Measurements

### A. In vitro 5-HT_{1A/B} Activity Measurements: 5-HT1_{A/B} cAMP HTRF assay (performed at Euroscreen, order no.: 5-HT1A:FAST-0500C, 5-HT1B: FAST-0501 C)

CHO-K1 cells expressing each human recombinant receptor (see Table 1) grown prior to the test in media without antibiotic were detached by gentle flushing with PBS-EDTA, recovered by centrifugation and resuspended in assay buffer. Dose response curves are performed in parallel with the reference compounds.

For agonist tests (96well): 12 µl of cells were mixed with 6 µl of the test compound at increasing concentrations and 6 µl of forskolin then incubated 30 min at room temperature. After addition of the lysis buffer and 1 hour incubation, cAMP concentrations were estimated, according to the manufacturer specification, with the HTRF kit (Cisbio).

### Material

Assay buffer consisted of: 5 mM KCI, 1.25 mM MgSO₄, 124 mM NaCl, 25 mM HEPES, 13.3 mM Glucose, 1.25 mM KH₂PO₄, 1.45 mM CaCl2, and 0.5 g/I BSA. PBS-EDTA contained 137 mM NaCl, 2.7 mM KCI, 10 mM Na₂HPO₄, 2 mM KH₂PO₄ and 5 mM EDTA. Cell lines used were derived from CHO-K1 stably expressing the human receptors needed for the pharmacological testing (see Table 1). 5-Carboxamidotryptamine (5-CT) was used as reference agonist.

**Table 1:**

| Receptor | Accession Number | Cell line | Reference agonist |
|---|---|---|---|
| 5-HT_{1A} | NP_000515.2 | CHO-K1 | 5-CT |
| 5-HT_{1B} | NP_000854.1 | CHO-K1 | 5-CT |
| 5-HT_{2B} | NP_00858.2 | CHO-K1 | Mianserin |

### B. 5-HT_{2B} assay (performed at Euroscreen, order no.: FAST-0506I):

The 5-HT_{2B} [00136] IP-One HTRF assay was conducted with recombinant cell lines, receptor accession numbers, cellular background and reference compounds are shown in **Table 1.**

Cells expressing human recombinant 5-HT_{2B} (FAST-506I) grown to mid-log phase in culture media without antibiotics will be detached with PBS-EDTA, centrifuged and resuspended in medium without antibiotics buffer. 20,000 cells will be distributed in a 96 well plate and incubated overnight at 37°C with 5% CO₂.

For agonist testing, the medium will be removed and 20 µl of assay buffer plus 20 µl of test compound or reference agonist will be added in each well. The plate will be incubated for 60 min at 37°C with 5% CO₂.

For antagonist testing, 20 µl of test compound or reference antagonist is added and the plate is incubated for 15 min. at 37°C in a humidified atmosphere of 95% air with 5% CO₂, then 20 µl of reference agonist at a final concentration corresponding to the historical EC₈₀ is added. The plate is incubated for 60 min at 37°C with 5% CO₂.

IP1-D2 reagent and anti-IP1 cryptate reagents will be then dispensed in the wells and DeltaF percentage values will be then calculated following the manufacturer (CisBio) recommendations.

### Quality Control

On each day of experimentation, reference compounds were tested at several concentrations in duplicate (n=2) to obtain a dose-response curve and an estimated EC₅₀ value.

Reference values thus obtained for the test were compared to historical values obtained from the same receptor and used to validate the experimental session. A session was considered as valid only if the reference value was found to be within a 0.5 logs interval from the historical value. For replicate determinations, the maximum variability tolerated in the test was of +/-20% around the average of the replicates.

Dose-response data from test compounds were analyzed with XLfit (IDBS) software using nonlinear regression applied to a sigmoidal dose-response model (4 Parameter Fit).

Agonist activity of test compounds is expressed as a percentage of the activity of the reference agonist at a saturating concentration.

### Results:

OPC-14523 showed partial agonism (~60% efficacies) at 5-HT_{1A} and 5-HT_{1B} (26 and 7 nM, respectively) (see Figure 1). Furthermore, OPC-14523 showed antagonistic activity at 5-HT_{2B} (160 nM).

### EXAMPLE 2: Effect of OPC-14523 on L-DOPA Induced Dyskinesia in the 6-hydroxydopamine (6-OHDA)-lesioned rat model of Parkinson's disease

### Materials and Methods

### Animals

Male Sprague Dawley rats (Elevage Janvier, Le Genest Saint Isle, France) weighing between 220 and 250 g at the beginning of the study are used in these experiments. They are housed under a 12-hr light/dark cycle with free access to standard pelleted food and tap water. Animal treatment and experimental procedures are in accordance with Animal Health regulations and are approved by local ethical committees.

### Dopamine-denervating lesions

Dopamine-denervating lesions are performed on rats anaesthetized with a 5:1 mixture of ketamine and xylazine (1 ml/kg, i.p.). All rats receive unilateral injection of 6-hydroxydopamine (6-OHDA-HCl) (4 µg/µL in 0.02% ascorbate-saline, in total 16µg/4 µl) into the right ascending DA fibre bundle at the following coordinates (in mm relative to bregma and the dural surface): A = - 4.4, L = -1.2, V = -8.3, tooth bar -2.3. Injections are performed at the rate of 1 µL/min (allowing an additional 3 min before retracting the needle) using a 10-µL Hamilton microsyringe with a 26-gauge steel cannula.

### Experimental Design and Drug Treatment

At 4 weeks post-lesion, rats are treated for 3 weeks (15 days in total, weekends excluded) with a single daily i.p. injection of 6 mg/kg of L-DOPA mixed with 15 mg/kg of the peripheral DOPA-decarboxylase inhibitor benserazide hydrochloride or with saline (vehicle controls). L-DOPA and benserazide are dissolved in a physiological saline solution. Chronic treatment with this dose of L-DOPA has been shown to induce gradual development of dyskinetic-like movements in 6-OHDA-lesioned rats. After ca. 3 weeks of the daily treatment, rats are administered 30 minutes before the evaluation of abnormal involuntary movement (AIM)s with different doses of OPC-14523 (3 and 10 mg/kg, dissolved in 20% HPBCD/distilled water, p.o.), followed by L-DOPA/benserazide (L-DOPA 6 mg/kg, benserazide 15 mg/kg), i.p., 20 minutes before the beginning of the test.

### Behavioral Test

In order to evaluate the severity of LID, AIMs are recorded every second day as described by Cenci et al. (1998). Cenci MA, Lee CS, Björklund A. L-DOPA-induced dyskinesia in the rat is associated with striatal overexpression of prodynorphin- and glutamic acid decarboxylase mRNA. Eur J Neurosci 1998 10:2694-2706. Repetitive movements affecting the side of the body contralateral to the lesion that could not be ascribed to any normal behavioural pattern are classified into four different subtypes: locomotive AIMs, i.e., increased locomotion with contralateral side bias; axial dystonia, i.e., contralateral twisted posturing of the neck and upper body; orolingual AIMs, i.e., stereotyped jaw movements and contralateral tongue protrusion; and forelimb dyskinesia, i.e., repetitive jerks of the contralateral forelimb, sometimes combined with grabbing movements of the paw. Each rat is scored on a severity scale from 0 to 4 based on its frequency and persistence (1 = occasional; 2 = frequent; 3 = continuous but interrupted by sensory distraction; 4 = continuous, severe and not interrupted by sensory distraction). The axial, orolingual and forelimb (AOL) AIMs are presented together as a mean (mean AIM score) per time point. Only rats with a dyskinesia severity ≥ grade 2 in two of the abnormal involuntary movements (axial and /or limb and /or orolingual), resulting in a cumulative abnormal involuntary movement (AIM) score ≥ 40 over the two consecutive selection sessions, are included in further experiments. In this experiment, L-DOPA (6 mg/kg + Benserazide 15mg/kg) is injected with vehicle or in combination with OPC-14523 at 3 and 10 mg/kg.

### Statistical Analysis

A signed-rank test is used for the evaluation of within-subject comparison of the cumulative data over the total time of 3 hours. A two-way ANOVA is used to evaluate the significance of the time course of LID. A Post hoc Tukey test is performed where appropriate.

### Results

**Figures 2a** and **2b** illustrate the effect of increasing doses of OPC-14523 on AIM scores in 6-OHDA-lesioned rats. The data are expressed as number of AIMs. * indicates a significant difference with p<0.05 between L-DOPA - vehicle-treated animals (ANOVA).

This result demonstrates that OPC-14523 (10 mg/kg) strongly reduced LID, showing an efficacy comparable to Eltoprazine (-60%, data not shown) and greater than Amantadine (-25%; data not shown))

The present invention is not to be limited in scope by the specific embodiments described herein. Indeed, various modifications of the invention in addition to those described herein will become apparent to those skilled in the art from the foregoing description. Such modifications are intended to fall within the scope of the appended claims.

All patents, applications, publications, test methods, literature, and other materials cited herein are hereby incorporated by reference to the extent permitted under the respective patent law and patent regulations.

## Claims

1. A composition comprising a therapeutically effective amount of a 4-substituted 1-aryl-piperazine selected from OPC-14523 and OPC-34712, particularly OPC-14523, or a pharmaceutically acceptable salt thereof for use in the treatment or prevention of L-DOPA-induced dyskinesia.

2. The composition according to claim 1, wherein the pharmaceutically acceptable salt is selected from a monohydrochloride; and a monomethanesulfonate, particularly a monohydrochloride.

3. The composition according to claim 1 or 2, wherein OPC-14523 or OPC-34712 is for administration in a range from about 5 mg to about 75 mg/day, or in a range from about 5 mg to about 60 mg/day, or in a range from about 5 mg to about 50 mg/day, or in a range from about 5 mg to about 40 mg/day, or in a range from about 5 mg to about 20 mg/day, or in a range from about 5 mg to about 15 mg/day, particularly wherein OPC-14523 or OPC-34712 is for administration in a range from about 10 mg to about 15 mg/day.

4. The composition according to any of the preceding claims, wherein the free base of OPC-14523 or OPC-34712 or a pharmaceutically acceptable salt thereof is for administration once a day, twice a day (b.i.d.), or three times a day.

5. The composition according to claim 4, wherein the free base of OPC-14523 or OPC-34712 or a pharmaceutically acceptable salt thereof is for administration twice a day (b.i.d.), particularly wherein the daily dosage for said administration twice a day (b.i.d.) is split into a first dose of about 55 to 65% of the total daily dosage amount, and a second dose comprising the remaining total daily dosage amount.

6. The composition according to any of the preceding claims, wherein the subject to be treated suffers from a movement disorder, particularly wherein the movement disorder is selected from parkinsonism, restless legs syndrome (RLS), and Chorea Huntington, particularly parkinsonism.

7. The composition according to claim 6, wherein the parkinsonism is idiopathic (PD).

8. The composition according to claim 7, wherein the subject is undergoing long-term treatment with L-DOPA therapy indicated for the treatment of PD.

9. The composition according to claim 6 or 7, wherein the subject is undergoing treatment with dopamine receptor agonists.

10. The composition according to any of claims 1 to 9, wherein said treatment or prevention comprises the additional administration with at least one additional pharmaceutical agent, particularly wherein the additional pharmaceutical agent(s) is/are selected from the group of (i) an agent which has been shown to be effective for the treatment of PD, and (ii) a decarboxylase inhibitor, particularly selected from benserazide and carbidopa.

11. The composition according to any of the preceding claims, wherein the free base of OPC-14523 or OPC-34712 or the pharmaceutically acceptable salt thereof is in the form of an oral formulation.

12. The composition according to any of the preceding claims, wherein said therapeutically effective amount of the free base of OPC-14523 or OPC-34712 or a pharmaceutically acceptable salt thereof is administered at least about 10 minutes before the administration of a therapeutically effective amount of L-DOPA.

13. The composition according to any of the preceding claims, wherein said method or composition is for the prevention of L-DOPA-induced dyskinesia, wherein the free base of OPC-14523 or OPC-34712 or a pharmaceutically acceptable salt thereof is administered to a subject receiving L-DOPA, wherein said subject is still free of symptoms of L-DOPA-induced dyskinesia, and the treatment with said composition is started at the latest 9 years after the beginning of said L-DOPA treatment.

14. The composition of claim 13, wherein said subject receives L-DOPA as first-line treatment of Parkinson's disease, particularly wherein said subject receives L-DOPA either in combination with a dopamine agonist or as a second-line treatment of Parkinson's disease.

15. The composition of any one of claims 1 to 14, wherein said treatment comprises administration of an increased dosage of L-DOPA compared to the dosage used by said subject without the administration of OPC-14523 or OPC-34712, particularly wherein said therapeutically effective amount of OPC-14523 or OPC-34712 is selected such that the L-DOPA-induced dyskinesia caused by said increased dosage of L-DOPA is kept at, or below, the level which was present in said subject without the administration of said therapeutically effective amount of OPC-14523 or OPC-34712.
